# EUROPEAN PATENT APPLICATION

(11) **EP 4 803 092 A1**
(43) Date of publication of application: **09.09.2026**
(21) Application number: 26160600.8
(22) Date of filing: 25.02.2026
(51) Int. Cl.: A61K 36/185, A23L 33/105, A61K 9/00, A61P 13/04

(54) **PUNICA GRANATUM AND TERMINALIA CHEBULA EXTRACTS FOR THE TREATMENT AND PREVENTION OF RENAL CALCULOSIS**

(30) Priority: 27.02.2025 IT 202500003921
(71) Applicant: Difass International S.p.A., 47853 Coriano (RN) (IT)
(72) Inventor: AGOSTINI, Alida, 47853 Coriano (RN) (IT); GIORGINI, Davide, 47039 Savignano sul Rubicone (FC) (IT)
(74) Representative: Bianchetti & Minoja with Trevisan & Cuonzo IPS SRL

(57) **Abstract**

Disclosed are oral compositions comprising a synergistic association of *Punica granatum* fruit extract and *Terminalia chebula* fruit extract in mixture with suitable carriers and excipients. The compositions are useful for the prevention and treatment of renal lithiasis.

## Description

The invention relates to a synergistic association of *Punica granatum* fruit extract and *Terminalia chebula* fruit extract useful for the prevention and treatment of renal lithiasis.

### Background of the Invention

Kidney stones, or renal nephrolithiasis, is a very widespread and dangerous clinical condition, because it increases the risk of chronic kidney disease.

In recent years, no significant progress has been made in scientific research on therapies and in the understanding of the pathophysiology of kidney stones. This disease remains a challenge for both the healthcare system and patients to this day.

Epidemiology varies according to geographical, socioeconomic and climatic factors. Age, sex, diet and lifestyle are known to influence the prevalence and incidence of the disease; for example, obesity and metabolic syndrome are recognized as risk factors, while diabetes, cardiovascular diseases, fractures and chronic kidney diseases are favoured by kidney stones as well as representing a risk factor. The prevalence of kidney stones increases with age. The highest prevalence, equal to 19.7%, was found in male individuals aged over 80 years, followed by 18.8% in men aged between 60 and 79 years, 11.5% in men aged between 40 and 59 years and 5.1% in men aged between 20 and 39 years. Men have a higher prevalence (NHANES (National Health and Nutrition Examination Survey) data from 2007 to 2010), 10.6% compared to 7.1% among women. A more recent NHANES study (2015-2016) reports a prevalence of 13.0% in men and 9.8% in women. This gender gap between men and women seems to be narrowing. According to NHANES 2017-2018 data, the male prevalence is even higher but rather stable, while the female prevalence shows a continuous increase. Calcium oxalate continues to be the dominant component of kidney stones globally. Over the past three decades, the prevalence of kidney stones has increased worldwide [Stamatelouet al., 2023].

Not only the frequency, but also the recurrence rates of nephrolithiasis are increasing because of the lack of effective therapies and methods. This increase could be attributed to the increasing incidence of obesity, a significant risk factor for kidney stones. Genetics, nutrition and some medical problems are additional risk factors. Dietary interventions are, therefore, essential for the prevention of kidney stones.

A recently published bibliometric analysis shows substantial growth in research in nutrition and kidney stones, reaching a peak in 2021. Research productivity in this area was particularly high in the United States, with Italy, China, Germany and India actively contributing. The research topics mainly concern: gender differences in kidney stones, the link between an increase in the consumption of acids in the diet and the development of calcium oxalate kidney stones and the connection between the ketogenic diet and the appearance of kidney stones. These results generally indicate a growing interest in nutrition and kidney stones. Continued research in these areas may significantly improve the prevention and treatment of renal calculosis [Zyoud et al., 2024].

The formation of kidney stones is a gradual and complex process, to which several causal factors contribute. In the clinical literature it is classically described as a process in two distinct phases: initiation and growth. The precipitation and subsequent crystallization of the lithogenic solutes are the first steps in the formation of the stones and depend on the urinary supersaturation of the solutes present. The calculations may have different chemical composition. Those containing calcium are by far the most common (75-90%), while struvite (ammonium and magnesium phosphate) and uric acid and cystine are much less frequent.

The causes of urolithiasis can be varied and contribute to varying degrees to influence its formation and clinical manifestation. These include: low urine volume, diet rich in animal proteins and oxalates, dehydration, hyperparathyroidism, renal tubular acidosis, family history of calculus, bariatric surgery, anatomical conditions that favour urinary stasis and urinary tract infections.

Small stones are generally asymptomatic, while bladder stones cause pain. The passage of kidney stones into the urinary tract (ureters, renal pelvis) may cause symptoms of obstruction (low back pain, renal colic), infection and bleeding.

Since the etiopathogenesis is multifactorial, also the prophylaxis contemplates the use of different agents able to act specifically on the different phases of the formation of the stones. As an expulsion therapy, in addition to the increase in fluid intake, alpha-blockers and calcium channel blockers, ureteral stent and percutaneous nephrostomy tube can be used. Techniques such as extracorporeal shock wave lithotripsy and endoscopic techniques are used to remove the stone.

The recurrence rate of secondary stone formation is estimated to be 10-23% per year, 50% in 5-10 years and 75% in 20 years of the patient [Moe 2006]. In particular, in patients who have formed a calcium stone for the first time, there is a risk of forming a second stone equal to 15% at 1 year, 40% at 5 years and 80% at 10 years. Therefore, prophylactic management is of paramount importance for managing urolithiasis.

Diuretics and citrate salts are used to reduce hypercalciuria and hyperoxaluria (the main causes of lithiasis). The latter, in particular, in addition to their manageability, safety of use and low costs, are widely used for their proven ability to sequester calcium in urine. In particular, the citrate-calcium complex is more soluble than the oxalate-calcium complex, thus inhibiting crystal agglomeration and subsequent precipitation. The pharmacological approach to the treatment of urate stones is based on molecules such as allopurinol, whose effectiveness is limited by adverse effects.

Scientific research is focused on the investigation of the etiopathogenetic mechanisms of renal lithiasis, whose understanding will guide the choices of prophylactic interventions.

In recent decades there have been significant advances and innovations in medical-surgical procedures to effectively address urolithiasis. However, the prevalence and incidence of urolith formation has increased since the last century worldwide. Epidemiological data suggest that, at least in part, this is due to changes in dietary habits.

It is unanimously recognized that nutritional management is the best preventive strategy against urinary lithiasis. Dietary advice aims to reduce most lithogenic risk factors by reducing urine supersaturation, primarily for calcium oxalate, calcium phosphate, and uric acid. High salt intake, consumption of red meat and consumption of foods rich in oxalates, such as certain vegetables and nuts, have been associated with an increased risk of urolithiasis due to their contribution to increased levels of calcium and oxalate in the urine. The Mediterranean diet, characterized by a high intake of fruits, vegetables, whole grains, and olive oil, and moderate consumption of fish and poultry, has been linked to a lower risk of urolithiasis [Zyoud et al, 2024].

Several medicinal plants have been proposed as medicines by virtue of their antilithiasic activity. In particular, the activity of aqueous extracts of fruits of *Terminalia chebula* [Tayal et al., 2012; Pawar et al., 2012] and *Punica granatum* [Rathod et al., 2012] has been described.

Although the utility of food plants, herbal supplements and phytochemicals has been well established in the treatment and prevention of diseases, mainly in urolithiasis, pharmacological studies and scientific evidence are not yet convincing [Nirumand et al, 2018; Oswal et al, 2023].

### Description of the invention

It has now been found that the fruit extracts of *Punica granatum* and *Terminalia chebula,* associated with each other, determine synergistic effects of inhibition of the nucleation of calcium oxalate crystals.

The present invention therefore relates to compositions for oral use comprising *Punica granatum* extract and *Terminalia chebula* extract useful for the treatment and prophylaxis of renal lithiasis.

The compositions of the invention determine an improved inhibitory activity of the formation of calcium oxalate stones, without significant side effects, overcoming the limits and criticalities of conventional botanical formulations characterized by the presence of numerous components and high daily doses that can compromise the adherence to the treatment both in the short and long term, as a consequence of the organoleptic-sensory characteristics and tolerability.

In addition to extracts, preferably dried, of fruits of *Punica granatum* and *Terminalia chebula* the compositions of the invention may advantageously contain citrate salts, in particular potassium citrate and magnesium citrate.

The fruit extracts of *Punica granatum* and *Terminalia chebula* are preferably in the 1:1 weight ratio while the ratios between the synergistic association of *Punica granatum* and *Terminalia chebula* and citrate salts range from 1:20 to 1:2, preferably from 1:10 to 1:3, more preferably from 1:6 to 1:5.

The compositions of the invention may contain other active ingredients such as extracts of *Phyllantus niruri, Ananas comosus, Parietaria officinalis, Ceterach officinarum, Arctostaphylos uva-ursi, Solidago virga-aurea,* probiotics, yeasts, bacterial lysates, postbiotics, prebiotics, oils, essences, polyphenols, bioflavonoids, vitamins, minerals. The dry extract of *Phyllantus niruri* is particularly preferred as an additional component.

The synergistic association of the invention can be formulated as a food or in the form of nutraceutical, pharmaceutical compositions or medical devices. The food may fall into the categories of fortified foods, foods for special medical purposes, food supplements, functional foods.

Examples of nutraceutical or pharmaceutical compositions include powders, micro granules, granules, microcapsules, tablets, lozenges, hard capsules, soft capsules, chewing gum, candies, soluble granules, aqueous or oily solutions, water-dispersible solutions, biphasic bottles, liquid stick packs, suspensions, oral drops.

Food, nutraceutical and pharmaceutical acceptable excipients are used for food, nutraceutical and pharmaceutical preparations.

The preparation of sachets/stick packs containing water-dispersible and/or orodispersible powders and/or granulates is carried out with conventional wet granulation techniques of the ingredients previously weighed and possibly sieved.

Solutions or suspensions are prepared by solubilizing or dispersing active ingredients and excipients in the aqueous liquid carrier generally in order of increasing solubility of the components. The solution or suspension obtained is then packaged in a special container.

In order to demonstrate the synergy of the association of the invention in inhibiting the crystallization of calcium oxalate salts, an experimental protocol was adopted as described by Hess et al. Most urinary stones have calcium oxalate as their main component, whose crystals can lead to the formation of aggregates, through a process called "nucleation", then to the formation of stone in the urinary tract. Two solutions were prepared: (i) 15 mM calcium chloride dihydrate (solution A); (ii) sodium oxalate (1.5 mM) (solution B). For both solutions, 200 mM NaCl was added and the pH was adjusted to 5.7 by adding 10 mM sodium acetate. Before use, the solutions were filtered through 0.22 µm filters. Crystal formation was triggered by mixing an equal volume of the two stock solutions. To evaluate the effectiveness of a substance in preventing or reducing crystal formation, the following experimental conditions were performed:
- stock solution A + stock solution B + distilled water corresponding to the negative control (crystal formation);
- stock solution A + stock solution B + tribasic potassium citrate solution 0.67 mg/mL corresponding to the positive control (no or reduced crystal formation);
- stock solution A + stock solution B + different concentrations of raw materials alone or mixtures thereof.

Crystal formation was assessed by turbidimetric analysis, recording absorbance at 620 nm every 15 seconds for 1 hour using a UV-visible spectrophotometer. The results were expressed as the percentage of nucleation inhibition and calculated using the following formula: [1- (slope _{tested condition}/slope _{NC})] x 100, where slope _{tested condition} represents the slope of the linear portion of the curve of the tested condition and slope _{NC} represents the slope of the linear portion of the negative control curve.

**Table 1: Experimental Conditions**

| | **Condition** | **Concentration (mg/mL)** |
|---|---|---|
| | **First phase** | **First phase** |
| P | *Punica granatum* dry fruit extract | 0.025; 0.25; 0.5 |
| C | *Terminalia chebula* dry fruit extract | 0.025; 0.25; 0.5 |
| B | *Terminalia Bellerica* dry fruit extract | 0.025; 0.25; 0.5 |

| | **Second phase** | **Second phase** |
|---|---|---|
| P | *Punica granatum* dry fruit extract | 0.012 |
| C | *Terminalia chebula* dry fruit extract | 0.012 |
| B | *Terminalia Bellerica* dry fruit extract | 0.012 |

| | **Third phase** | **Third phase** |
|---|---|---|
| P+C | *Punica granatum* dry fruit extract + *Terminalia chebula* dry fruit extract | 0.012 (each) |
| C+B | *Terminalia chebula* dry fruit extract + *Terminalia bellerica* dry fruit extract | 0.012 (each) |
| P+B | *Punica granatum* dry fruit extract + *Terminalia Bellerica* dry fruit extract | 0.012 (each) |
| P+C+B | *Punica granatum* dry fruit extract + *Terminalia chebula* dry fruit extract + *Terminalia bellerica* dry fruit extract | 0.012 (each) |

| | **Fourth phase** | **Fourth phase** |
|---|---|---|
| | Potassium citrate tribasic | 0.21 |
| | *Punica granatum* dry fruit extract + *Terminalia chebula* dry fruit extract + Citrate salts | *0.012 Punica granatum* dry fruit extract *+0.012 Terminalia chebula* dry fruit extract + 0.21 citrate salts |

**Table 2: results of nucleation inhibition (%). ^{at}T-test vs negative control;**

| ^{b}T-test vs positive control. | | |
|---|---|---|
| | **Extracts (mg/mL)** | **Inhibition of nucleation (%)** |
| P | *Punica granatum* dry fruit extract 0.025 | 68.33 ± 0.25^{ab} |
| P | *Punica granatum* dry fruit extract 0.25 | 71.19 + 0.45^{ab} |
| P | *Punica granatum* dry fruit extract 0.5 | 79.56 ± 0.72^{ab} |
| C | *Terminalia chebula* dry fruit extract 0.025 | 50.43 ± 0.41^{ab} |
| C | *Terminalia chebula* dry fruit extract 0.25 | 79.37 ± 0.63^{ab} |
| C | *Terminalia chebula* dry fruit extract 0.5 | 83.42 ± 0.27^{ab} |
| B | *Terminalia Bellerica* dry fruit extract 0.025 | 25.62 ± 5.24^{ab} |
| B | *Terminalia Bellerica* dry fruit extract 0.25 | 24.86 ± 2.54^{ab} |
| B | *Terminalia Bellerica* dry fruit extract 0.5 | 45.10 ± 1.56^{ab} |
| Ctr - | Negative control | 0.00 ± 39.73^{b} |
| Ctr + | Positive control | 85.23 ± 0.39^{a} |

A significant effect of inhibition of crystal nucleation appears from all samples tested with an expected dose-dependent activity. Considering the significant inhibition of all the chosen concentrations (first phase), the second phase was started with further lowered concentrations in order to be able to better appreciate potential synergistic effects of the components under study. In fact, it is desirable to use not too high amounts of the components for administration in humans, for better gastric and systemic tolerability, even in the long term, in order to support their use or compliance. The results of the inhibition of the nucleation of the calcium oxalate crystals of the individual extracts at very low concentrations (0.012 mg/ml) are shown in Table 3. All extracts show very similar percentages and significantly higher than the negative control, but an order of magnitude lower than the positive control.

**Table 3: results of nucleation inhibition (%) 0.012 mg/ml. ^{a}T-test vs negative control; ^{b}T-test vs positive control.**

| | **Extracts (mg/mL)** | **Inhibition of nucleation (%)** |
|---|---|---|
| M | *Punica granatum* dry fruit extract 0.012 | 9.10 ± 1.13^{ab} |
| C | *Terminalia chebula* dry fruit extract 0.012 | 9.97 ± 0.44^{ab} |
| B | *Terminalia Bellerica* dry fruit extract 0.012 | 8.60 ± 0.88^{ab} |
| Ctr - | Negative control | 0.00 ± 0.80^{b} |
| Ctr + | Positive control | 73.70 ± 0.45^{a} |

The results of nucleation inhibition by combining the different extracts together (third phase) are shown in Table 4.

**Table 4: Results of nucleation inhibition (%) of 0.012 mg/ml associations.**

| ^{a}T-test vs negative control; ^{b}T-test vs positive control. | | |
|---|---|---|
| | **Extracts (mg/mL)** | **Inhibition of nucleation (%)** |
| P+C | *Punica granatum* dry fruit extract 0.012 + *Terminalia chebula* dry fruit extract 0.012 | 71.79 ± 0.55^{ab} |
| C+B | *Terminalia chebula* dry fruit extract 0.012 + *Terminalia bellerica* dry fruit extract 0.012 | 40.87 ± 0.34^{ab} |
| P+B | *Punica granatum* dry fruit extract 0.012 + *Terminalia bellerica* dry fruit extract 0.012 | 46.20 ± 0.84^{ab} |
| P+C+B | *Punica granatum* dry fruit extract 0.012 + *Terminalia chebula* dry fruit extract 0.012 + *Terminalia bellerica* dry fruit extract 0.012 | 72.56 ± 0.55^{ab} |
| Ctr - | Negative control | 0.00 ± 3.78^{b} |
| Ctr + | Positive control | 80.91 ± 0.07^{a} |

A surprising and unexpected synergy emerges only for the association of *Punica granatum* and *Terminalia chebula* (P+C). The extracts of *Punica granatum* and *Terminalia chebula* at very low concentration (0.012 mg/ml) show a percentage of inhibition of the nucleation of calcium oxalate crystals similar to the positive control, to the percentages obtained at very high concentration of the individuals and to the P+C+B mix (sum of all three extracts with a final concentration of 0.036 mg/ml): 71.79 ± 0.55.

The unexpected effect of the P+C association is evident considering the efficiency in inhibiting the nucleation of the crystals of the individual extracts and their associations compared to the percentage value of the positive control of the respective experimental steps. The following formula was used for the comparison: $% Δ Ctr + = \left[\left(% inibizione Ctr+\right)-\left(% inibizione componente\right)\right] x 100 / % inibizione Ctr +$

Table 5 shows the % Δ Ctr+ of the relevant tests for the evaluation of the synergistic effect of *Punica granatum* and *Terminalia chebula:* individual components with a concentration of 0.012 mg/ml (same concentration used in the associations), individual components with a concentration of 0.025 mg/ml (concentration more than double that of the individual ones in association, but approximately equivalent to their sum), associations of the extracts (1+1), each with a concentration of 0.012 mg/ml. The unexpected synergy P+C is further and more detectable in the direct comparison with Ctr+: only 11.27% less activity than Ctr+, significantly lower than the albeit brilliant result of *Punica granatum* at more than double concentration (19.82%). Further evidence is given by the absence of a synergistic, albeit cumulative, effect of C+B and P+B.

**Table 5: Delta (Δ) percent vs positive control (Ctr+)**

| | **Extracts (mg/mL)** | **% Δ Ctr+** |
|---|---|---|
| P | *Punica granatum* dry fruit extract 0.025 | 19.82 |
| P | *Punica granatum* dry fruit extract 0.012 | 87.65 |
| C | *Terminalia chebula* dry fruit extract 0.025 | 40.83 |
| C | *Terminalia chebula* dry fruit extract 0.012 | 86.47 |
| B | *Terminalia Bellerica* dry fruit extract 0.025 | 69.94 |
| B | *Terminalia Bellerica* dry fruit extract 0.012 | 88.33 |
| P+C | *Punica granatum* dry fruit extract 0.012 + *Terminalia chebula* dry fruit extract 0.012 | 11.27 |
| C+B | *Terminalia chebula* dry fruit extract 0.012 + *Terminalia bellerica* dry fruit extract 0.012 | 49.49 |
| P+B | *Punica granatum* dry fruit extract 0.012 + *Terminalia bellerica* dry fruit extract 0.012 | 42.90 |

Citrate salts are known to inhibit the nucleation of calcium oxalate crystals, the main and most frequent responsible for renal lithiasis. In fact, citrates are used in different concentrations in products for the prevention of kidney stones. In order to highlight interference and/or additive or even synergistic effects with the P+C association, the following were tested and compared in the same experimental model:
- P+C (0.012 mg/ml + 0.012 mg/ml) + S (Citrate salts), wherein the citrate anion (AC) share was chosen within the following ratio range (P+C)/AC = 1:20 and (P+C)/AC = 1:2
- Ctr+
- Ctr-

As expected, P+C+S showed a complementary effect of the individuals, therefore in the absence of interference in the crystal formation inhibition activity, but without any synergistic effect with the citrate salts.

Examples of formulations of the invention are given below.

### Example 1: Water-dispersible sachet

| | mg/sachet |
|---|---|
| *Terminalia chebula* Retz | 100.00 |
| *Punica granatum L.* | 100.00 |
| Maltodextrin | 963.00 |
| Citric acid | 200.00 |
| Flavouring | 100.00 |
| Silica dioxide | 22.00 |
| Isomalt | 10.00 |
| Sucralose | 5.00 |
| TOTAL | 1,500.00 |

### Example 2: Water-dispersible sachet

| | mg/sachet |
|---|---|
| *Phyllantus niruri L.* | 150.00 |
| *Terminalia chebula* Retz | 100.00 |
| *Punica granatum L.* | 100.00 |
| Potassium citrate | 900.00 |
| Magnesium citrate | 650.00 |
| Maltodextrin | 736.50 |
| Citric acid | 200.00 |
| Flavouring | 100.00 |
| Silica dioxide | 45.00 |
| Isomalt | 10.00 |
| Sucralose | 5.00 |
| Polisorbate Tween 80 | 3.50 |
| TOTAL | 3,000.00 |

### Example 3: Water-dispersible sachet

| | mg/sachet |
|---|---|
| *Phyllantus niruri L.* | 150.00 |
| *Terminalia chebula* Retz | 100.00 |
| *Punica granatum L.* | 100.00 |
| Potassium citrate | 856.97 |
| Magnesium citrate | 777.20 |
| Bromelain | 167.80 |
| Sodium hyaluronate | 54.19 |
| Maltodextrin | 460.34 |
| Citric acid | 170.00 |
| Flavouring | 100.00 |
| Silica dioxide | 45.00 |
| Isomalt | 10.00 |
| Sucralose | 5.00 |
| Polisorbate Tween 80 | 3.50 |
| TOTAL | 3,000.00 |

### Example 4: orodispersible stick pack

| | mg/sachet |
|---|---|
| *Phyllantus niruri L.* | 75.00 |
| *Terminalia chebula* Retz | 50.00 |
| *Punica granatum L.* | 50.00 |
| Potassium citrate | 450.00 |
| Magnesium citrate | 325.00 |
| Maltodextrin | 774.25 |
| Flavouring | 50.00 |
| Citric acid | 30.00 |
| Silica dioxide | 17.00 |
| Sucralose | 2.00 |
| Polisorbate Tween 80 | 1.75 |
| TOTAL | 1,800.00 |

### Example 5

| | % w/v |
|---|---|
| *Phyllantus niruri L.* | 1.00% |
| *Terminalia chebula* Retz | 0.67% |
| *Punica granatum L.* | 0.67% |
| Potassium citrate | 12.06% |
| Glycerol | 31.22% |
| Sorbitol | 5.12% |
| Flavouring | 1.82% |
| Xanthan gum | 0.69% |
| Citric acid | 0.60% |
| Preservatives | 0.23% |
| Sucrester | 0.12% |
| Sweeteners | 0.03% |
| Water | as needed 100% |
| TOTAL | 100% |

### Bibliography

Hess B, Meinhardt U, Zipperle L, Giovanoli R, Jaeger P. Simultaneous measurements of calcium oxalate crystal nucleation and aggregation: impact of various modifiers. Urol Res 1995;21:231-238.
Moe OW, "Kidney stones: pathophysiology and medical management," <e Lancet, vol. 367, no. 9507, pp. 333-344, 2006.
Nirumand MC, Hajialyani M, Rahimi R, Farzaei MH, Zingue S, Nabavi SM, et al. Dietary plants for the prevention and Management of Kidney Stones: preclinical and clinical evidence and molecular mechanisms. Int J Mol Sci. (2018) 19:765.
Oswal M, Varghese R, Zagade T, Dhatrak C, Sharma R, Kumar D. Dietary supplements and medicinal plants in urolithiasis: diet, prevention, and cure. J Pharm Pharmacol. 2023;75(6):719-745.
Pawar l T. , Gayatri D. Gaikwad, Kavita S. Metkari, Kiran A. Tijore, Jaydip V. Ghodasara, Bhanudas S. Kuchekar Effect of Terminalia chebula fruit extract on ethylene glycol induced urolithiasis in rats. Biomedicine & Aging Pathology Volume 2, Issue 3, July-September 2012, Pages 99-103.
Rathod NR , Dipak Biswas, H.R. Chitme, Sanjeev Ratna, I.S. Muchandi, Ramesh Chandra d Anti-urolithiatic effects of Punica granatum in male rats. Journal of Ethnopharmacology. Volume 140, Issue 2, 27 March 2012, Pages 234-238
Stamatelou K, Goldfarb DS. Epidemiology of Kidney Stones. Healthcare (Basel). 2023 Feb 2;11(3):424.
Tayal S 1, S Duggal, P Bandyopadhyay, A Aggarwal, S Tandon, C Tandon. Cytoprotective role of the aqueous extract of Terminalia chebula on renal epithelial cells. Int Braz J Urol 2012 Mar-Apr;38(2):204-13;
Tracy, C.R.; Henning, J.R.; Newton, M.R.; Aviram, M.; Bridget Zimmerman, M. Oxidative stress and nephrolithiasis: A comparative pilot study evaluating the effect of pomegranate extract on stone risk factors and elevated oxidative stress levels of recurrent stone formers and controls. Urolithiasis 2014, 42, 401-408.
Zyoud SH, Abushamma F, Salameh H, Abushanab AS, Koni A, Taha AA, Al-Jabi SW, Shahwan M, Jairoun AA, Shakhshir MH. Exploring the nutritional landscape and emerging trends in kidney stone research: visualization and bibliometric analysis. Translational Medicine Communications (2024) 9:8.

## Claims

1. Oral compositions comprising a association of *Punica granatum* fruit extract and *Terminalia chebula* fruit extract in mixture with suitable carriers and excipients for use in the prevention and treatment of renal lithiasis.

2. Compositions for use according to claim 1 where the weight ratio between *Punica granatum* fruit extract and *Terminalia chebula* fruit extract is 1:1.

3. Compositions for use according to claim 1 or 2 comprising citrate salts, preferably potassium citrate and magnesium citrate.

4. Compositions for use according to any one of claims 1 to 3, wherein the ratios of *Punica granatum* plus *Terminalia chebula* to citrate anion range from 1:20 to 1:2, preferably from 1:10 to 1:3, more preferably from 1:6 to 1:5.

5. Compositions for use according to claims 1-4 comprising an extract of *Phyllantus niruri.*

6. Compositions for use according to any one of claims 1 to 5, further comprising one or more ingredients selected from extracts or powders of *Ananas comosus, Parietaria officinalis, Ceterach officinarum, Arctostaphylos uva-ursi, Solidago virga-aurea,* fermentates, probiotics, yeasts, bacterial lysates, postbiotics, prebiotics, oils, essences, polyphenols, bioflavonoids, vitamins, minerals.

7. Compositions for use according to one of claims 1 to 6 in the form of foods, powders, microgranules, granules, microcapsules, tablets, lozenges, hard capsules, soft capsules, chewing gum, candies, soluble granules, aqueous or oily solutions, water-dispersible solutions, two-phase bottles, liquid stick packs, suspensions, drops.
